(19) Europäisches Patentamt

European Patent Office

Office européen des brevets

(11) Veröffentlichungsnummer: **0 300 425 B1**

(12) **EUROPÄISCHE PATENTSCHRIFT**

(45) Veröffentlichungstag der Patentschrift: **21.07.93**

(51) Int. Cl.⁵: **C12N 15/67**

(21) Anmeldenummer: **88111602.4**

(22) Anmeldetag: **19.07.88**

Die Akte enthält technische Angaben, die nach dem Eingang der Anmeldung eingereicht wurden und die nicht in dieser Patentschrift enthalten sind.

(54) Verfahren zur Herstellung von Proteinen in löslicher Form.

(30) Priorität: **20.07.87 DE 3723992**

(43) Veröffentlichungstag der Anmeldung:
**25.01.89 Patentblatt 89/04**

(45) Bekanntmachung des Hinweises auf die
Patenterteilung:
**21.07.93 Patentblatt 93/29**

(84) Benannte Vertragsstaaten:
**AT BE CH DE ES FR GB GR IT LI LU NL SE**

(56) Entgegenhaltungen:
**EP-A- 159 123**
**EP-A- 192 988**

**Molecular and General Genetics vol.216, no.1,Januar 1989, Seiten 149-155; KOPETZKI, E et al.: "Control of formation of active soluble or inactive insoluble baker s yeastalphaglucosidase PI in Escherichia coli by induction and growth conditions"**

**BIOTECHNOLOGY vol. 6, März 1988, New York US, Seiten 291-294; Schein,C.H. & NOTEBORN, M.H: "Formation of soluble recombinant proteins in escherichia coli is favored by lower growth temperature"**

**BIOTECHNOLOGY LETTERS vol. 8, no.9, September 1986, Seiten 605-610; MIZUKAMI, TAMIO et al.: "Production of active human interferon beta in e.coli: IPreferential production by lower culture temperature"**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Kopetzki, Erhard, Dr. rer.nat.**
**Henle-Strasse 2**
**W-8122 Penzberg(DE)**
Erfinder: **Schumacher, Günther, Dr. rer.nat.**
**Kapellenweg 20**
**W-8139 Bernried(DE)**

(74) Vertreter: **Huber, Bernhard, Dipl.-Chem. et al Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 300 425 B1

**Beschreibung**

Die Erfindung betrifft ein Verfahren zur Expression von unter der Kontrolle eines induzierbaren Promotor stehender rekombinanter DNA in geeigneten Wirtszellen.

Durch rekombinante DNA-Technologie lassen sich viele Probleme bei der Produktion von Proteinen lösen oder wesentlich vereinfachen. Bei dieser Technologie wird die für das gewünschte Protein kodierende DNA-Sequenz (Gen) in einen Expressionsvektor integriert und die Proteinexpression über regulatorische Sequenzen, insbesondere über Promotoren, gesteuert.

Besonders geeignete Promotoren, mit denen hohe Ausbeuten an homologen und heterologen Proteinen erhalten werden können, sind solche, die in der Regel sowohl hohe als auch gleichzeitig kontrollierbare Aktivität zeigen. Derartige Promotoren sind für E. coli die natürlichen-, Hybrid- und Bakteriophagenpromotoren, wie beispielsweise der lac-, lacuv5-, trp-, tac-, trc-, rac-, phoA-, mgl-, $\lambda$-$P_L$-, $\lambda$-$P_R$-, $T_5$-, $T_7$- und SP6-Promotor.

Die Aktivität dieser Promotoren kann beispielsweise durch Induktoren (Substrate, Substratanaloga), die Temperatur oder die Menge an verfügbarer Bakteriophagen-RNA-Polymerase gesteuert werden. Hiermit kann beispielsweise erreicht werden, daß das heterologe Protein nur während bestimmter Zeiten der Fermentation, nämlich z. B. nicht in der Wachstumsphase der als Wirt für den Expressionsvektor verwendeten Mikroorganismen, exprimiert wird. Eine derartige Steuerung ist insbesondere dann vorteilhaft, wenn das heterologe Protein für die Wirtszelle toxisch ist. Zur Expression von heterologen Proteinen in Wirten wie E. coli, vor allem, wenn das Protein für E. coli toxisch ist, wird deshalb üblicherweise so verfahren, daß nach einer Anzuchtphase, in der sich der Promotor in einem reprimierten Zustand befindet, ein geeigneter Induktor zur vollständigen Aktivierung des Promotors zugesetzt wird. Der Promotor und damit die Expression des Fremdproteins, wird oft in der spätlogarithmischen bis frühstationären Wachstumsphase der Wirtszelle nach Erreichen einer geeignet hohen Zelldichte (Biomasse) induziert (Mitzukamie et al., Biotech. Lett. 8 (1986) 611-614). Bei Verwendung des lac-, lacuv5-, tac-, trc- und rac-Promotors wird als Induktor beispielsweise Isopropyl-$\beta$-D-thiogalactopyranosid (IPTG) in einer Konzentration von ca. 1 mM (Miller, Experiments in Molecular Genetics, Cold Spring Harbor Laboratory, Cold Spring Harbor, New York, 1972) zugesetzt. Die Expression von unter lac-Operator-Kontrolle stehendem Protein in Stämmen mit erhöhter lac-Repressorsynthese (lacI$^q$) steigt mit zunehmender Induktorkonzentration (IPTG: 0, 2 und 20 mM) (Luck et al., DNA 5 (1986) 21-28). Die Repression des trp-Promotors durch Tryptophan wird aufgehoben durch Entfernung des Corepressors Tryptophan (tryptophanfreies Medium) und/oder durch Induktion mit 3,$\beta$-Indolylacrylsäure (IAA) in einer Konzentration von 20 bis 50 mg/L (Smith et al., Gene 32 (1984) 321 - 327; Mosteller et al., Symp. Quant. Biol. 35 (1970) 461-466).

Es hat sich jedoch gezeigt, daß dieses Verfahren nicht für alle homologen und heterologen Proteine zu befriedigenden Ausbeuten an Protein der gewünschten Eigenschaften wie Löslichkeit, natürliche Konformation und gegebenenfalls biologische Aktivität, führen. Während für manche enzymatisch aktive Proteine, wie z. B. $\beta$-Galactosidase, $\beta$-Lactamase, Chloramphenicolacetyltransferase, (CAT) Creatinase, Penicillinacylase Löslichkeit und hohe Enzymaktivität nach Expression in E. coli erreicht werden, erhält man unter Anwendung der gleichen Bedingungen und der gleichen Promotoren bei anderen Proteinen nur geringe Mengen bzw. überhaupt kein lösliches Protein oder gar biologisch aktives Produkt exprimiert. Derartige Effekte werden beispielsweise bei $\alpha$-Glucosidase, Prochymosin, den schweren und leichten Ketten von Antikörpern, Immunglobulinen, Urokinase, t-PA, Interferon, Wachstumshormon, Fusionsproteinen aus $\beta$-Galactosidase und Hiv-Antigenen bzw. Humaninsulin-Polypeptiden beobachtet (Marston, Biochem. J. 240 (1986) 1 - 12).

Es konnte jedoch gezeigt werden, daß diese Proteine durchaus in großen Mengen in den entsprechenden Zellextrakten vorhanden sind, jedoch unlöslich sind oder/und keine biologische Aktivität aufweisen.

Als Ursache für die geringe Expression von löslichem, insbesondere aktivem Protein in E. coli werden beispielsweise die Denaturierung des Fremdproteins zu refractile bodies (inclusion bodies), d. h. zu unlöslichen Proteinaggregaten, sowie eine Proteolyse des Fremdgenprodukts durch zelleigene Proteasen diskutiert (Carriers et al., Trends in Biotechnology 1 (1983) 109-113). Um hier Abhilfe zu schaffen, wird von Mitzukamie et al., Biotech. Lett. 8 (1986) 605-610 für die Expression von Interferon-$\beta$ in E. coli unter Kontrolle des trp-Promotors vorgeschlagen, die Fermentation bei 20 °C durchzuführen, da bei dieser Temperatur die Ausbeute an aktivem IFN-$\beta$ ansteigt. Nachteilig an diesem Verfahren ist jedoch, daß Fermentationszeiten von mindestens 4 Tagen benötigt werden, um Aktivitäten zu erhalten, für welche sich die Aufarbeitung lohnt.

Außerdem wurde die Verwendung von Wirtszellen, denen das für die Spaltung des Genproduktes verantwortlich gemachte proteolytische System fehlt, vorgeschlagen. Beispielsweise kann eine lon-Mutante von E. coli als Wirtszelle verwendet werden. Derartige Mutanten sind defizient bezüglich einer der im Wildtyp vorkommenden Protease. Da allerdings in E. coli mindestens 7 weitere Proteasen vorliegen (Swamy et al., J. Bacteriol. 149 (1982) 1027-1033), ist das Verfahren nur dann geeignet, wenn das zu exprimierende Protein von diesen anderen Proteasen nicht gespalten wird. Außerdem ist die Auswahl von geeigneten Wirtszellen sehr beschränkt.

Weiter wurde vorgeschlagen, die Expression durch die Erhöhung der Kopienzahl des Expressionsvektors so weit zu steigern, daß die Proteasen durch eine riesige Menge an gebildetem Protein überschwemmt werden und demzufolge ihre Wirkung im Verhältnis nur bei einem geringen Prozentsatz des Proteins entfalten. Der Nachteil dieser Methode ist jedoch, daß die Expression innerhalb von nur 1 bis 2 Generationen immens gesteigert werden muß und nicht längere Zeit durchgeführt werden kann (Trends in Biotechnology 1; (1983) 109-113).

Aufgabe der vorliegenden Erfindung war es daher, die geschilderten Nachteile des Standes der Technik zu vermeiden, und die Herstellung von löslichen, insbesondere biologisch aktiven Proteinen zu ermöglichen.

Gelöst wird die Aufgabe durch ein Verfahren zur Expression von unter der Kontrolle eines induzierbaren Promotors stehender rekombinanter DNA in geeigneten Wirtszellen, welches dadurch gekennzeichnet ist, daß man zur Herstellung von Proteinen, die unter üblichen gentechnologischen Herstellungsbedingungen in unlöslicher Form anfallen, in löslicher Form die Induktion des Promotors auf weniger als 10% der maximalen Induktion im Vergleich mit einem Standardsystem limitiert und damit die Transkriptionsrate der für das Protein kodierenden DNA in mRNA entsprechend begrenzt, worin als Standardsystem die Expression eines unter den gegebenen Bedingungen in löslicher, aktiver Form anfallenden Proteins unter der Kontrolle desselben Promoters auzusehen ist.

Durch die Limitation der Induktion des Promotors gelingt es, die Bildung von refractile bodies weitgehend zu verhindern und stattdessen lösliches, insbesondere aktives Protein in großen Mengen zu erzeugen. Ohne eine solche Limitierung der Expressionsgeschwindigkeit führt vollständige Induktion zwar zu einer sehr schnellen Bildung von Protein, das dann jedoch in Form von refractile bodies oder als inaktive Proteine oder Proteinfragmente vorliegt.

Bevorzugte Ausführungsformen sind in den Unteransprüchen charakterisiert. Beim erfindungsgemäßen Verfahren wird die Induktion des Promotors bevorzugt auf weniger als 5%, besonders bevorzugt auf weniger als 1% der maximalen Induktion im Vergleich mit einem Standardsystem begrenzt.

Als Standardsystem ist zum einen ein Vektor, in dem das zu exprimierende Fremdgen durch ein anderes hetero-oder homologes Gen ersetzt wurde, welches in diesem System (gleiche Wirtszelle) lösliches aktives Protein exprimiert, wobei dieses "Standardgen" dann natürlich unter der Kontrolle des gleichen Promotors wie das Fremdgen stehen muß, geeignet. Mögliche Standardgene sind z. B. $\beta$-Lactamase, CAT, oder Creatinase. Als weiteres Standardsystem wäre auch die Wirtszelle selbst (ohne Vektor) geeignet, wenn sie ein Standardgen unter vergleichbarer Kontrolle des zur Verwendung vorgesehenen Promotors bereits chromosomal integriert enthält (siehe Beispiel 2a, $\beta$-Galactosidase-Gen von E.coli im natürlich vorkommenden lac-Operon).

Geeignet zur Expression der rekombinanten DNA sind alle Wirtszellen, in denen die Expression einer unter der Kontrolle eines induzierbaren Promotors stehenden rekombinanten DNA möglich ist. Bevorzugt wird die Expression in E. coli, besonders bevorzugt in E. coli-Stämmen, welche ein lacI$^q$Gen tragen, durchgeführt. Ein Beispiel hierfür ist E. coli, DSM 2102, welcher mit Plasmid pePA 119 (DSM 3691P) transformiert ist. Dieser Stamm wird im folgenden mit ED82-I$^q$ bezeichnet. Dabei erfolgt die Induktion günstigerweise in der logarithmischen Wachstumsphase. Als Promotoren sind für das erfindungsgemäße Verfahren in E. coli natürliche, Hybrid- oder Bakteriophagenpromotoren geeignet. Bevorzugt werden der lac-, lacuv5-, trp-, tac-, trc-, rac-, phoA-, mgl-, $\lambda$-P$_L$-, $\lambda$-P$_R$-, T$_5$-, T$_7$- oder SP6-Promotor verwendet.

Eine maximale Induktion der $\beta$-Galactosidase in E. coli Wildtypzellen (lacI$^+$Z$^+$Y$^+$) wird mit einer Induktorkonzentration von 0,1 bis 1 mM IPTG erreicht (Knippers, R.: Molekulare Genetik, Georg Thieme Verlag, Stuttgart, New York (1985)). In Anwesenheit einer intakten Lactose-Permease (lacY$^+$) wird IPTG aktiv in die Zelle transportiert und dort mehr als 50fach angereichert, so daß intrazellulär eine Induktorkonzentration von mehr als 5 mM existiert (Miller, Experiments in Molecular Genetics, Cold Spring Harbour Laboratory, Cold Spring Harbour, New York (1972).

Gemäß einer bevorzugten Ausführungsform der Erfindung wird eine limitierte Induktion durch Herabsetzen der effektiven Induktorkonzentration am Wirkort (intrazellulär) auf kleiner als 10 %, bevorzugt kleiner als 5 %, insbesondere kleiner als 1 % im Vergleich mit einem, wie oben definierten Standardsystem, bewirkt. Die maximale Induktion ist dann erreicht, wenn nach Verdoppelung der Induktorkonzentration die spezifische Enzymaktivität des Standards weniger als 15 % zunimmt.

Die Limitation der Induktion kann beispielsweise durch begrenzte Zugabe von Induktor bewirkt werden. Bei Verwendung des lac-Promotors oder einem davon abgeleiteten Promotor wird als Induktor IPTG, bevorzugt in einer Konzentration von weniger als 0,01 mM zugegeben.

Gemäß einer weiteren bevorzugten Ausführungsform erfolgt die Begrenzung der Induktion durch Limitierung des aktiven Transports des Induktors durch die Zellmembran. Ein defektes Transportsystem kann beispielsweise dadurch erhalten werden, daß im Lactose-Permease-Gen (lacY) von E. coli eine Mutation angebracht wird. Als Induktor geeignet sind in diesem Fall beispielsweise IPTG oder Lactose. Bei defektem Transportsystem entspricht dann die intrazelluläre IPTG-Konzentration der extrazellulären Konzentration (Miller, Experiments in Molecular Genetics, Cold Spring Harbor Laboratory, Cold Spring Harbor, New York (1972)). Lactose kann trotz defekter Lactosepermease noch in geringem Umfang über das Thiomethylgalactosidasel und Arabinose Transportsystem in die Zelle aufgenommen werden (Rotman et al., J.Mol.Biol. 36, 247-260 (1968); Messer, J.Bacteriol. 120, 266-272 (1974)).

Bevorzugt verwendet man den lac- oder einen davon abgeleiteten Promotor in einer Wirtszelle, welche eine Mutation in diesem Lactose-Permease-Gen (lacY) trägt.

Besonders bevorzugt verwendet man als Wirtszellen, welche ein defektes Lactose-Permease-Gen und zusätzlich das lac-I$^q$Gen enthalten, insbesondere E.coli ED82-I$^q$, DSM 2102, transformiert mit Plasmid DMS 3691P, welches das lac-I$^q$Gen trägt. Zur Induktion wird Lactose dem Kulturmedium in einer Konzentration kleiner 1 % zugesetzt. Die im Medium vorhandene Lactose wird kaum verstoffwechselt, sie reicht aber zur Induktion des Promotors aus.

Ebenso kann die Begrenzung der Induktion durch Zugabe von Induktoren erreicht werden, die bei der Fermentation verstoffwechselt werden und damit ihre induzierende Wirkung verlieren.

Eine weitere Ausführungsform der Begrenzung der Induktion im erfindungsgemäßen Verfahren besteht darin, die Aktivität von Promotoren durch Beeinflussung der Effektor-Promotor-Wechselwirkung zu kontrollieren. Dabei ist nicht die Konzentration des Induktors am Wirkort entscheidend, sondern seine Affinität zum Repressor oder umgekehrt. Eine Herabsetzung der Induktor-Repressor-Wechselwirkung kann beispielsweise durch Verwendung eines Mikroorganismus erreicht werden, der ein Repressor-Mutanten-Gen besitzt, das für ein Repressor-Protein mit einer veränderten Repressor-Induktor-Bindungskonstante codiert. Bevorzugt wird in einem solchen Verfahren der lac-Promotor sowie ein Mikroorganismus, der eine lac-Repressormutanten-Gen (lacI$^s$) trägt, verwendet. Eine weitere Möglichkeit ist beispielsweise die Verwendung von Promotoren, die eine CAP-Stelle besitzen, wobei die Affinität des Katabolit-Aktivator-Proteins zum Promotor herabgesetzt wird.

Der erfindungsgemäße Effekt der Begrenzung der mRNA-Bildung oder Induktion des Promotors kann durch zusätzliche Maßnahmen, die das Wachstum der Zellen bei der Fermentation und den Zeitpunkt der Induktorzugabe betreffen, gesteigert werden. So gelingt die Expression von löslicher aktiver α-Glucosidase durch eine sehr langsame Induktion des tac-Promotors und zusätzliche steigernde Maßnahmen, die das Wachstum beeinflussen.

Die Wachstumsgeschwindigkeit von Zellen läßt sich beispielsweise durch die Art des Mediums (Voll- und Minimalmedien, wie z. B. DYT, LB, M9CAA und M9), die C-Quelle (wie z. B. Zucker, Glycerin und Pepton), die N-Quelle (wie z. B. $(NH_4)_2SO_4$, Aminosäuren und Pepton) den pH-Wert des Mediums, die Temperatur, die Menge an verfügbarem Sauerstoff, sowie den Stammhintergrund (Mutationen) beeinflussen. Ein verändertes Wachstum kann auch durch mehrere, sich überlagernde Effekte bewirkt werden. So wird z. B. durch Abbau von fermentierbaren C-Quellen das Medium stark angesäuert (pH 4,8), wodurch sich das Wachstum verlangsamt.

Die folgenden Beispiele und Figuren sollen die Erfindung weiter erläutern.

Fig. 1: Nucleotid-Sequenz und davon abgeleitete Aminosäuresequenz von α-Glucosidase pI

Fig. 2: Konstruktion des α-Glucosidase pI Plasmids pKK 177-3/GLUCPI zur Expression in E.coli

**Beispiele**

Expression von α-Glucosidase in E. coli:

Zur Manipulation von DNA wurden Standardmethoden benutzt, wie sie bei Maniatis et al. (1982) in Molecular Cloning, Cold Spring Harbor Laboratory, Cold Spring Harbor, New York 11724, beschrieben sind. Die verwendeten molekularbiologischen Reagenzien wurden nach Angaben des Herstellers eingesetzt.

**Beispiel 1**

Konstruktion des alpha-Glucosidase pI-Expressionsvektors (pKK177-3/GLUCPI)

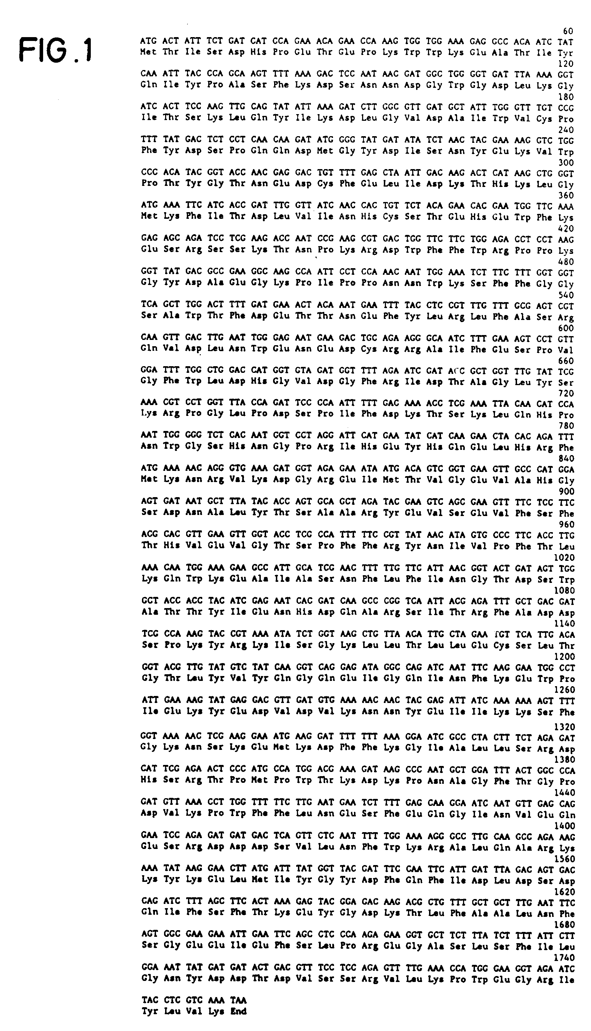

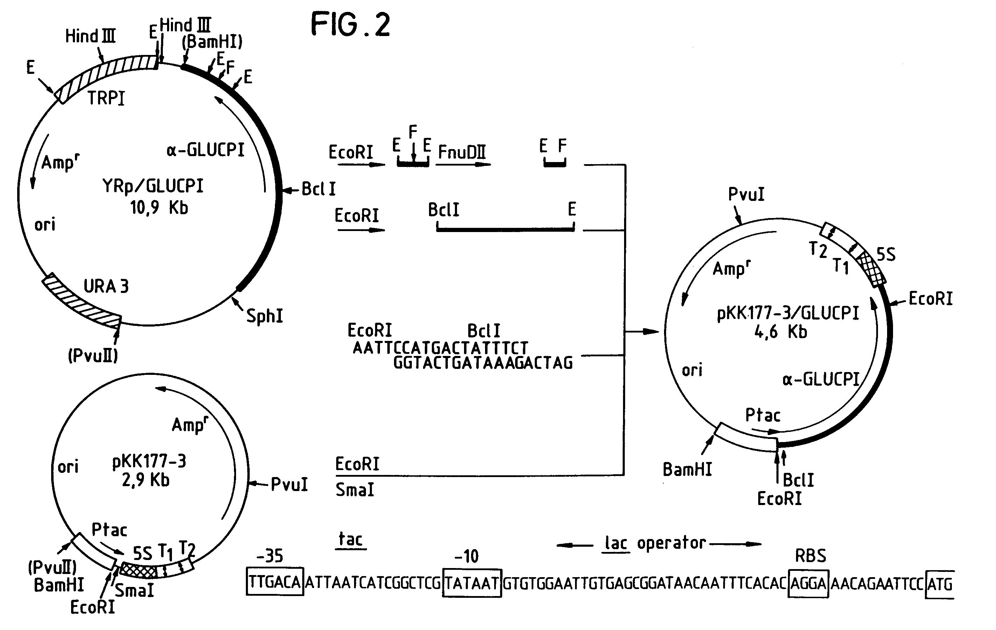

Das Strukturgen der alpha-Glucosidase pI (Fig. 1) aus Bäckerhefe wird über einen Adapter (korrekte Verbindung zwischen Promotor und N-Terminus) und zwei α-Glucosidase codierende DNA-Fragmente des Plasmids YRp/GLUCPI, DSM 4173P, zusammengesetzt (Fig. 2). Die 3′-untranslatierte Region der alpha-Glucosidase aus Hefe ist hierbei bis auf 25 bp entfernt.

Hierzu wird das Plasmid YRp/GIUCPI mit EcoRI und BclI verdaut und das BclI/EcoRI-Fragment (1,7 kb) sowie das EcoRI-Fragment (ca. 0,3 kb) isoliert. Das EcoRI-Fragment wird mit FnuDII nachgespalten und das EcoRI/FnuDII-Fragment (0,13 kb) isoliert.

Der Vektor pKK177-3, DSM 3062, wird mit EcoRI und SmaI gespalten. In das hierbei entstandene Vektorfragment (2,85 kb) werden das BclI/EcoRI-Fragment, das EcoRI/FnuDII-Fragment sowie das synthetische DNA-Fragment

```
5'-AATTCCATGACTATTTCT     -3'
3'-     GGTACTGATAAAGACTAG-5'
```

ligiert.

Die gewünschte Konstruktion wurde auf 5-Brom-4-chlorindolyl-alpha-D-glucopyranosid-Indikatorplatten (alphaXG1, 40 mg/l) und 1 mM IPTG anhand von geringer alpha-Glucosidase-Aktivität in ED82-I$^q$ identifiziert und isoliert.

Durch Restriktionsanalyse wird der korrekte Zusammenbau des $\alpha$-GLUCPI-Gens bestätigt. Das Plasmid trägt die Bezeichnung pKK177-3/GLUCPI. Der Wirtsstamm ED82-I$^q$ besitzt unter Testbedingungen keine alpha-Glucosidase-Aktivität.

**Beispiel 2**

Expression von Hefe-alpha-Glucosidase pI in E. coli unter Standardbedingungen

Zur heterologen Expression der alpha-Glucosidase pI aus Hefe wurde der E. coli K-12-Stamm ED82-I$^q$, welcher den Vektor pKK177-3/GLUCPI enthält, benutzt. Die Experimente wurden in Rollerkultur (20 ml-Reagenzglas mit 5 ml Medium) in LB-Medium (Maniatis et al., Molecular Cloning, Cold Spring Harbor Laboratory (1982)) mit 40 mg/l Ampicillin bei 37°C durchgeführt. Die Kultur wurde mit 50 µl Übernachtkultur angeimpft und beim Erreichen einer Zelldichte von OD$^{550}$ zwischen 0,5 und 0,6 mit IPTG (5 mM Endkonzentration) induziert. Nach 2 bis 3 Stunden Inkubation bei Raumtemperatur werden Proben der Kultur (0,5 ml) genommen, zentrifugiert, der Zellkuchen mit 10 mM Phosphatpuffer pH 6,8 gewaschen und zu Aufbewahrung tiefgekühlt.

Bestimmung von $\alpha$-Glucosidase und $\beta$-Galactosidase

Der Zellkuchen wird mit 0,25 ml 10 mM Phosphatpuffer pH 6,8 und 1 mM EDTA resuspendiert und die Zellen durch Ultraschallbehandlung aufgeschlossen. Nach Zentrifugation wird die aktive $\alpha$-Glucosidase im Überstand durch Zugabe von 2 mM p-Nitrophenyl-2-D-Glucopyranosid (PNPG) in 0,1 M Phosphatpuffer pH 6,8 bei 25°C photometrisch bestimmt (405 nm).

Die Berechnung der spezifischen Aktivität erfolgte entsprechend der Microbinret-Methode (Methods Enzymol. 3 (1957) 696-704) mit Rinderserumalbumin als Standard. Spezifische Aktivitäten werden angegeben als Nanomol hydrolysiertes Substrat pro Minute und Milligramm Protein.

Die Aktivität der $\beta$-Galactosidase wurde in Analogie zum $\alpha$-Glucosidase-Test mit 2-Nitrophenyl-$\beta$-galactopyranosid anstelle von PNPG als Substrat bestimmt. Die chromosomal kodierte $\beta$-Galactosidase in ED82-I$^q$ diente zur Kontrolle des Induktionsverlaufes des lac-Operons.

**Beispiel 3**

a) Abhängigkeit der Ausbeute an aktivem Protein (alpha-Glucosidase pI und $\beta$-Galactosidase) in Abhängigkeit von der Induktionsdauer und der IPTG-Induktorkonzentration.

Es wurde wie unter den im Beispiel 2 beschriebenen Bedingungen gearbeitet, wobei die Induktorkonzentration (IPTG) und die Anzuchtdauer variiert wurden. Die Ergebnisse zeigt die Tabelle I.

Die Ergebnisse zeigen, daß die spezifische Aktivität der $\beta$-Galactosidase (interne Kontrolle) mit steigender Induktorkonzentration und steigender Induktionsdauer stetig zunimmt. Im Gegensatz zur $\beta$-Galactosidase erreicht die spezifische Aktivität der $\alpha$-Glucosidase ein Maximum bei einer Induktorkonzentration von 0,01 mM an IPTG.

b) Abhängigkeit der Ausbeute an aktivem Protein (alpha- Glucosidase und $\beta$-Galactosidase) in Abhängigkeit von der Lactose-Induktorkonzentration.

Es wurde wie unter den im Beispiel 2 beschriebenen Bedingungen gearbeitet, wobei die Induktorkonzentration (Lactose) variiert wurde. Die Ergebnisse sind in Tabelle II wiedergegeben.

Das Ergebnis zeigt, daß die $\beta$-Galactosidase (interne Kontrolle) durch eine Lactosekonzentration von 2% voll induziert wird. Im Gegensatz dazu erreicht die alpha-Glucosidase bei gleicher Induktorkonzentration nur eine spezifische Aktivität von 5% der maximal erreichbaren spezifischen Enzymaktivität.

## Beispiel 4

Abhängigkeit der Ausbeute an aktivem Protein (alpha-Glucosidase und $\beta$-Galactosidase) vom pH-Wert und von der Induktorkonzentration IPTG.

Es wurde wie unter den im Beispiel 2 beschriebenen Bedingungen gearbeitet, wobei der pH-Wert zum Zeitpunkt der Induktion durch Zugabe von Tris/HCl- bzw. Phosphatpuffer (Endkonzentration 0,1 M) eingestellt wurde und eine Induktorkonzentration von 0,01 mM IPTG (Tabelle III a) bzw. 0,5 % Lactose (Tabelle III b) verwendet wurde. Die Ergebnisse sind in Tabelle III a und III b wiedergegeben.

Es zeigt sich, daß bei dem für die Anzucht von E. coli optimalen pH-Bereich (7,0 bis 7,5) überraschenderweise die geringsten Ausbeuten an aktivem Protein erhalten werden. Die optimalen pH-Bereiche liegen zwischen 4,8 und 5,6 sowie zwischen 7,5 und 8,5. Darüberhinaus wird die Enzymaktivität um den Faktor 8 gegenüber alleiniger Induktion mit Lactose (0,5 %) gesteigert. (Tabelle II, Zeile 4 gegenüber Tabelle III b, Zeile 5).

## Beispiel 5

Abhängigkeit der Ausbeute an aktivem Protein (alpha-Glucosidase und $\beta$-Galactosidase) von der Anzuchttemperatur.

Es wurde wie unter den im Beispiel 2 beschriebenen Bedingungen gearbeitet, wobei die Temperatur und der Induktor IPTG (0,01 mM) bzw. Lactose (0,5%) variiert wurden. Die Ergebnisse sind in Tabelle IV dargestellt.

Die Ergebnisse zeigen, daß die spezifische Aktivität der $\beta$-Galactosidase (interne Kontrolle) nicht von der Anzuchtstemperatur beeinflußt wird. Überraschenderweise steigt die spezifische Aktivität der alpha-Glucosidase bei niedrigen Anzuchttemperaturen.

## Beispiel 6

Abhängigkeit der Ausbeute von aktivem Protein (alpha-Glucosidase und $\beta$-Galactosidase) vom Medium und der Anzuchtdauer.

Es wurde wie unter den im Beispiel 2 beschriebenen Bedingungen gearbeitet, wobei das Medium variiert und als Induktor 0,5% Lactose verwendet wurde (siehe Tabelle V).

## Beispiel 7

Abhängigkeit der Ausbeute an aktivem Protein (alphaGlucosidase und $\beta$-Galactosidase) von der C-Quelle.

Es wurde wie unter den im Beispiel 2 beschriebenen Bedingungen gearbeitet, wobei die C-Quelle (2% Endkonzentration) und der Induktor (Lactose 0,5%) zum Zeitpunkt der Induktion zugegeben wurden. Nach 2,5 und 6 Stunden wurde die spezifische Aktivität der alpha-Glucosidase und $\beta$-Galactosidase bestimmt (siehe Tabelle VI).

**Beispiel 8**

Bevorzugte Anzuchtbedingungen von aktiver alpha-Glucosidase

Zur Synthese von aktiver $\alpha$-Glucosidase wurde ED82-I$^q$ mit plasmidkodierter $\alpha$-Glucosidase in LB-Medium bzw. Minimalmedium bei 37 °C bis zu einer $OD^{550}$ von 0,4 bis 0,6 angezogen. Danach wurde die Kultur abgekühlt (20 bis 30 °C), mit Lactose (Endkonzentration 0,5%) induziert und entweder eine C-Quelle (Endkonzentration 1 bis 2%; vorzugsweise Glycerin und/oder Maltose) zugegeben oder der pH-Wert mit Phosphatpuffer (0,1M) auf pH 4,8 bis 5,5 herabgesetzt und die Zellen bei 20 bis 30 °C bis zu einer Zelldichte $OD^{550}$ von 3 bis 5 angezogen (siehe Tabelle VII).

Tabelle I

Ausbeute an aktivem Protein (mU/mg Protein)

Fermentationsdauer (min)

| Induktor IPTG (mM) | 40 | | 85 | | 145 | | 450 | |
|---|---|---|---|---|---|---|---|---|
| | α-Gluc | ß-Gal | α-Gluc | ß-Gal | α-Gluc | ß-Gal | α-Gluc | ß-Gal |
| 0 | 175 | 1. | 132. | 2. | 140. | 12. | 11. | 17. |
| 0,01 | 193 | 2. | 194. | 6. | 156. | 15. | 8. | 46. |
| 0,033 | 120 | 6. | 152. | 16. | 123. | 27. | 7. | 57. |
| 0,066 | 83 | 12. | 113. | 27. | 102. | 33. | 7. | 85. |
| 0,1 | 82 | 24. | 102. | 36. | 106. | 47. | 6. | 140. |
| 0,33 | 60 | 48. | 86. | 67. | 102. | 100. | 12. | 281. |
| 0,66 | 48 | 50. | 77. | 83. | 100. | 116. | 10. | 334. |
| 1. | 58 | 66. | 80. | 96. | 88. | 117. | 9. | 384. |
| 3. | 62 | 76. | 78. | 95. | 90. | 116. | 9. | 405. |
| 6. | 68 | 74. | 84. | 114. | 97. | 135. | 6. | 436. |

Tabelle II

| Induktor Lactose (%) | Ausbeute an aktivem Protein (mU/mg Protein) | |
|---|---|---|
| | $\alpha$-Gluc | $\beta$-Gal |
| 0 | 54 | 1 |
| 0,05 | 110 | 5 |
| 0,1 | 134 | 9 |
| 0,5 | 199 | 36 |
| 1. | 223 | 38 |
| 2. | 13 | 1.080 |

Tabelle III

| a) pH-Wert Induktor IPTG 0,01 (mM) | Ausbeute an aktivem Protein (mU/mg Protein) | |
|---|---|---|
| | $\alpha$-Gluc | $\beta$-Gal |
| 8,7 | 3 | - |
| 8,0 | 168 | 1 |
| 7,0 | 53 | 1 |
| 6,3 | 79 | 1 |
| 6,0 | 264 | 1 |
| 5,6 | 384 | 1 |
| b) Induktor Lactose (0,5 %) | $\alpha$-Gluc | $\beta$-Gal |
| 8,0 | 179 | 14 |
| 7,0 | 346 | 17 |
| 6,0 | 867 | 17 |
| 5,5 | 969 | 19 |
| 5,0 | 1629 | 5 |

Tabelle IV

| Ausbeute an aktivem Protein (mU/mg Protein) Temperatur °C | | | | | | | | |
|---|---|---|---|---|---|---|---|---|
| Induktor | 22 | | 27 | | 32 | | 37 | |
| | $\alpha$-Gluc. | $\beta$-Gal | $\alpha$-Gluc. | $\beta$-Gal | $\alpha$-Gluc. | $\beta$-Gal | $\alpha$-Gluc. | $\beta$-Gal |
| IPTG (mM) 0,06 | n.d. | n.d. | 1500 | 16 | 935 | 24 | 125 | 75 |
| Lactose (%) 0,5 | 2200 | 6 | 1560 | 5 | 1050 | 9 | 460 | 22 |
| n.d. nicht gemessen | | | | | | | | |

T a b e l l e    V

Ausbeute an aktivem Protein
(mU/mg Protein)
Anzuchtdauer (h)

| Medium | 3 | | 6 | | 20 | |
|---|---|---|---|---|---|---|
| | α-Gluc. | ß-Gal | α-Gluc. | ß-Gal | α-Gluc. | ß-Gal |
| LB | 600 | 4 | 1500 | 13 | 4 | 124 |
| M9CAA** | 30 | - | 110 | 4 | 2000 | 113 |
| M9* | 10 | - | 20 | - | 3000 | 83 |

\* M9-Minimalmedium          \*\* M9CAA-Minimalmedium

pro Liter:                     M9 mit 0,5 % Casaminosäuren

$Na_2 HPO_4$      6 g
$KH_2 PO_4$      3 g
NaCl       0,5 g
$NH_4 CL$      1 g
$MgSO_4\ 7H_2O$   1 mM
Thiamin      1 mg/L
Glucose      0,2 %

Tabelle VI

| Ausbeute an aktivem Protein (mU/mg Protein) Anzuchtdauer (h) | | | | |
|---|---|---|---|---|
| C-Quelle | 2,5 | | 6 | |
| | α-Gluc. | β-Gal | α-Gluc. | β-Gal |
| Glucose | 414 | - | 649 | 3 |
| Maltose | 1535 | 7 | 1982 | 7 |
| Glycerin | 833 | 12 | 2838 | 14 |
| Maltose/Glycerin | 1312 | 12 | 1744 | 9 |

T a b e l l e   VII

| Medium | C-Quelle (%) | Anzuchtzeit (h) | Temperatur (°C) | Induktor Lactose (%) | Ausbeute an aktivem Protein (mU/mg Protein) | |
|---|---|---|---|---|---|---|
| | | | | | α-Gluc. | β-Gal |
| LB | Glycerin | 20 | 27 | 0,5 | 5500 | - |
| LB | Glycerin | 20 | 37 | 0,5 | 5000 | - |
| LB | - | 6 | 27 | 0,5 | 4000 | 110 |
| M9 | - | 20 | 37 | 0,5 | 3500 | 110 |
| M9 | Glycerin | 20 | 37 | 0,5 | 4000 | n.d. |
| M9 | Gly/Mal | 20 | 37 | 0,5 | 3900 | n.d. |

n. d. nicht gemessen

**Patentansprüche**

1. Verfahren zur Expression von unter der Kontrolle eines induzierbaren Promotors stehender rekombinanter DNA in geeigneten Wirtszellen,

**dadurch gekennzeichnet,**

daß man zur Herstellung von Proteinen, die unter üblichen gentechnologischen Herstellungsbedingungen in unlöslicher Form anfallen, in löslicher Form die Induktion des Promotors auf weniger als 10% der maximalen Induktion im Vergleich mit einem Standardsystem limitiert und damit die Transkriptionsrate der für das Protein kodierenden DNA in mRNA entsprechend begrenzt, worin als Standardsystem die Expression eines unter den gegebenen Bedingungen in löslicher, aktiver Form anfallenden Proteins unter der Kontrolle desselben Promotors anzusehen ist.

2. Verfahren nach Anspruch 1, **dadurch gekennzeichnet,** daß man die Induktion des Promotors auf weniger als 5%, insbesondere weniger als 1% der maximalen Induktion begrenzt.

3. Verfahren nach Anspruch 1 und 2,
**dadurch gekennzeichnet,**
daß die Expression in E. coli-Stämmen, die ein lac I$^q$-Gen enthalten, durchgeführt wird.

4. Verfahren nach Anspruch 3, **dadurch gekennzeichnet,** daß die Expression in E. coli, DSM 2102, durchgeführt wird.

5. Verfahren nach Anspruch 1 bis 4, **dadurch gekennzeichnet,** daß man die Induktion in der logarithmischen Wachstumsphase durchführt.

6. Verfahren nach Anspruch 3, **dadurch gekennzeichnet,** daß bei Expression in E. coli als Promotor einer der natürlichen, bekannten Hybrid- oder Bakteriophagen-Promotoren verwendet wird.

7. Verfahren nach Anspruch 6, **dadurch gekennzeichnet,** daß der lac-, lacuv5, trp-, tac-, trc-, rac-, phoA-, mgl-, λ-$P_L$-, λ-$P_R$-, $T_5$-, $T_7$- oder SP6-Promotor verwendet wird.

8. Verfahren nach Anspruch 1 bis 7,
**dadurch gekennzeichnet,**
daß die Begrenzung der Induktion des Promotors durch limitierte Zugabe von Induktor bewirkt wird.

9. Verfahren nach einem der vorhergehenden Ansprüche, **dadurch gekennzeichnet,** daß man den lac-Promotor, oder einen davon abgeleiteten Promotor und als Induktor Isopropyl-$\beta$-D-thio-galactopyranosid (IPTG) in einer Konzentration von weniger als 0,01 mM verwendet.

10. Verfahren nach Anspruch 9, **dadurch gekennzeichnet,** daß man als Induktor Lactose in einer Konzentration von weniger als 1 % verwendet.

11. Verfahren nach Anspruch 1 bis 6,
**dadurch gekennzeichnet,**
daß man die Begrenzung der Induktion durch Limitierung des aktiven Transports des Induktors durch die Zellmembran bewirkt.

12. Verfahren nach Anspruch 9, **dadurch gekennzeichnet,** daß man den lac- oder einen davon abgeleiteten Promotor in einer Wirtszelle verwendet, welche eine Mutation im Lactosepermease (lacY)-Gen trägt und als Induktor Lactose oder IPTG verwendet.

13. Verfahren nach Anspruch 12, **dadurch gekennzeichnet,** daß man als Wirtszellen Zellen von E. coli ED82-I$^q$, DSM 2102, welche das Plasmid DSM 3691P enthalten, verwendet.

14. Verfahren nach Anspruch 1 bis 7,
**dadurch gekennzeichnet,**
daß man die Induktion des Promotors durch Beeinflussung der Effektor-Promotor-Wechselwirkung begrenzt.

15. Verfahren nach Anspruch 14, **dadurch gekennzeichnet,** daß man einen Microorganismus verwendet, der eine Repressormutante mit veränderter Repressor-Induktor-Bindungskonstante trägt.

**16.** Verfahren nach Anspruch 15, **dadurch gekennzeichnet,** daß man den lac-Promotor sowie einen Mikroorganismus, der ein lac-Repressormutanten-Gen (lacl$^s$) enthält, verwendet.

**17.** Verfahren nach Anspruch 14, **dadurch gekennzeichnet,** daß man Promotoren verwendet, die eine Chloramphenicolacetyltransferase(CAP)-Bindungsstelle besitzen, und die Affinität des Katabolit-Aktivator-Proteins zum Promotor herabsetzt.

**18.** Verfahren nach Anspruch 1 bis 17,
**dadurch gekennzeichnet,**
daß man die Produktion von löslichem Protein durch Steuerung des Zellenwachstums durch entsprechende Fermentationsbedingungen und den Zeitpunkt der Induktorzugabe, zusätzlich zu den die Induktion limitierenden Maßnahmen, steigert.

## Claims

**1.** Process for the expression in suitable host cells of recombinant DNA standing under the control of an inducible promotor, characterised in that, for the production in soluble form of proteins which, under usual gene-technological production conditions, are obtained in insoluble form, one limits the induction of the promotor to less than 10% of the maximum induction in comparison with a standard system and therewith correspondingly limits the transcription rate of the DNA coding for the protein into mRNA, wherein the expression of a protein obtained under the given conditions in soluble, active form under the control of the same promotor is to be regarded as a standard system.

**2.** Process according to claim 1, characterised in that one limits the induction of the promotor to less than 5%, especially to less than 1%, of the maximum induction.

**3.** Process according to claim 1 and 2, characterised in that the expression is carried out in E. coli strains which contain a lac I$^q$ gene.

**4.** Process according to claim 3, characterised in that the expression is carried out in E. coli, DSM 2102.

**5.** Process according to claim 1 to 4, characterised in that one carries out the induction in the logarithmic growth phase.

**6.** Process according to claim 3, characterised in that, in the case of expression in E. coli, as promotor there is used a natural known hybrid or bacteriophage promotor.

**7.** Process according to claim 6, characterised in that the lac, lacuv5, trp, tac, trc, rac, phoA, mgl, λ-P$_L$, λ-P$_R$, T$_5$, T$_7$ or SP6 promotor is used.

**8.** Process according to claim 1 to 7, characterised in that the limitation of the induction of the promotor is brought about by limited addition of inducer.

**9.** Process according to one of the preceding claims, characterised in that one uses the lac promotor or a promotor derived therefrom and, as inducer, isopropyl-ß-D-thiogalactoside (IPTG) in a concentration of less than 0.01 mM.

**10.** Process according to claim 9, characterised in that, as inducer, one uses lactose in a concentration of less than 1%.

**11.** Process according to claim 1 to 6, characterised in that one brings about the limitation of the induction by limitation of the active transport of the inducer through the cell membrane.

**12.** Process according to claim 8, characterised in that one uses the lac promotor or a promotor derived therefrom in a host cell which carries a mutation in the lactose permease (lacY) gene and uses lactose or IPTG as inducer.

**13.** Process according to claim 12, characterised in that, as host cells, one uses cells of the E. coli ED82-I^q, DSM 2102, which contains the plasmid DSM 3691P.

**14.** Process according to claim 1 to 7, characterised in that one limits the induction of the promotor by influencing the effector-promotor exchange action.

**15.** Process according to claim 14, characterised in that one uses a micro-organism which carries a repressor mutant with changed repressor-inducer binding constant.

**16.** Process according to claim 15, characterised in that one uses the lac promotor, as well as a microorganism which contains a lac repressor mutant gene (lacI^s).

**17.** Process according to claim 14, characterised in that one uses promotors which possess a chloramphenicol acetyltransferase (CAP) binding site and the affinity of the catabolite-activator protein to the promotor is reduced.

**18.** Process according to claim 1 to 17, characterised in that one increases the production of soluble protein by control of the cell growth by appropriate fermentation conditions and the point of time of the inducer addition, in addition to the measures limiting the induction.

**Revendications**

**1.** Procédé d'expression d'un ADN recombiné qui est sous le contrôle d'un promoteur inductible dans des cellules hôtes appropriées, caractérisé en ce que, pour la préparation sous forme soluble de protéines qui, dans les conditions de préparation par génie génétique habituelles, apparaissent sous forme insoluble, on limite l'induction du promoteur à moins de 10% de l'induction maximale par rapport à un système standard et on limite ainsi le taux de transcription en ARNm de l'ADN codant pour la protéine, dans lequel le système standard est constitué par l'expression d'une protéine qui apparaît sous forme Soluble et active dans les conditions données, sous le contrôle du même promoteur.

**2.** Procédé selon la revendication 1, caractérisé en ce qu'on limite l'induction du promoteur à moins de 5%, en particulier à moins de 1%, de l'induction maximale.

**3.** Procédé selon les revendications 1 et 2, caractérisé en ce que l'expression est accomplie dans des souches de E. coli qui contiennent un gène lac I^q.

**4.** Procédé selon la revendication 3, caractérisé en ce que l'expression est accomplie dans E. coli, DSM 2102.

**5.** Procédé selon les revendications 1 à 4, caractérisé en ce que l'on accomplit l'induction dans la phase de croissance logarithmique.

**6.** Procédé selon la revendication 3, caractérisé en ce que, pour l'expression dans E. coli, on utilise comme promoteur l'un des promoteurs naturels, hybrides ou bactériophagiques connus.

**7.** Procédé selon la revendication 6, caractérisé en ce que l'on utilise le promoteur lac, lacuv5, trp, tac, trc, rac, phoA, mgl, λ-P_L, λ-P_R, T_5, T_7 ou SP6.

**8.** Procédé selon les revendications 1 à 7, caractérisé en ce que la limitation de l'induction du promoteur est réalisée par addition limitée d'inducteur.

**9.** Procédé selon l'une des revendications précédentes, caractérisé en ce que l'on utilise le promoteur lac, ou un promoteur qui en dérive, et comme inducteur l'isopropyl-ß-D-thiogalactopyranoside (IPTG) en une concentration inférieure à 0,01 mM.

**10.** Procédé selon la revendication 9, caractérisé en ce que l'on utilise comme inducteur le lactose en une concentration inférieure à 1%.

**11.** Procédé selon les revendications 1 à 6, caractérisé en ce que l'on réalise la limitation de l'induction en limitant le transport actif de l'inducteur à travers la membrane cellulaire.

**12.** Procédé selon la revendication 9, caractérisé en ce que l'on utilise le promoteur lac ou un promoteur qui en dérive dans une cellule hôte qui porte une mutation dans le gène de la lactose perméase (lacY) et l'on utilise comme inducteur le lactose ou IPTG.

**13.** Procédé selon la revendication 12, caractérisé en ce que l'on utilise comme cellules hôtes des cellules de E. coli ED82-I$^q$, DSM 2102, qui contiennent le plasmide DSM 3691P.

**14.** Procédé selon les revendications 1 à 7, caractérisé en ce qu'on limite l'induction du promoteur en influençant l'interaction effecteur-promoteur.

**15.** Procédé selon la revendication 14, caractérisé en ce que l'on utilise un micro-organisme qui porte un mutant de répresseur présentant une constante de liaison répresseur-inducteur modifiée.

**16.** Procédé selon la revendication 15, caractérisé en ce que l'on utilise le promoteur lac et un micro-organisme qui contient un gène de mutant de répresseur lac (lacI$^s$).

**17.** Procédé selon la revendication 14, caractérisé en ce que l'on utilise des promoteurs qui possèdent un site de liaison de la chloramphénicolacétyltransférase (CAP) et qui abaissent l'affinité de la protéine activatrice de catabolite pour le promoteur.

**18.** Procédé selon les revendications 1 à 17, caractérisé en ce que l'on augmente la production de protéine soluble en commandant la croissance cellulaire par des conditions de fermentation correspondantes et par le moment de l'addition d'inducteur, en plus des dispositions limitant l'induction.

**FIG.1**

```
                                                              60
ATG ACT ATT TCT GAT CAT CCA GAA ACA GAA CCA AAG TGG TGG AAA GAG GCC ACA ATC TAT
Met Thr Ile Ser Asp His Pro Glu Thr Glu Pro Lys Trp Trp Lys Glu Ala Thr Ile Tyr
                                                              120
CAA ATT TAC CCA GCA AGT TTT AAA GAC TCC AAT AAC GAT GGC TGG GGT GAT TTA AAA GGT
Gln Ile Tyr Pro Ala Ser Phe Lys Asp Ser Asn Asn Asp Gly Trp Gly Asp Leu Lys Gly
                                                              180
ATC ACT TCC AAG TTG CAG TAT ATT AAA GAT CTT GGC GTT GAT GCT ATT TGG GTT TGT CCG
Ile Thr Ser Lys Leu Gln Tyr Ile Lys Asp Leu Gly Val Asp Ala Ile Trp Val Cys Pro
                                                              240
TTT TAT GAC TCT CCT CAA CAA GAT ATG GGG TAT GAT ATA TCT AAC TAC GAA AAG GTC TGG
Phe Tyr Asp Ser Pro Gln Gln Asp Met Gly Tyr Asp Ile Ser Asn Tyr Glu Lys Val Trp
                                                              300
CCC ACA TAC GGT ACC AAC GAG GAC TGT TTT GAG CTA ATT GAC AAG ACT CAT AAG CTG GGT
Pro Thr Tyr Gly Thr Asn Glu Asp Cys Phe Glu Leu Ile Asp Lys Thr His Lys Leu Gly
                                                              360
ATG AAA TTC ATC ACC GAT TTG GTT ATC AAC CAC TGT TCT ACA GAA CAC GAA TGG TTC AAA
Met Lys Phe Ile Thr Asp Leu Val Ile Asn His Cys Ser Thr Glu His Glu Trp Phe Lys
                                                              420
GAG AGC AGA TCC TCG AAG ACC AAT CCG AAG CGT GAC TGG TTC TTC TGG AGA CCT CCT AAG
Glu Ser Arg Ser Ser Lys Thr Asn Pro Lys Arg Asp Trp Phe Phe Trp Arg Pro Pro Lys
                                                              480
GGT TAT GAC GCC GAA GGC AAG CCA ATT CCT CCA AAC AAT TGG AAA TCT TTC TTT GGT GGT
Gly Tyr Asp Ala Glu Gly Lys Pro Ile Pro Pro Asn Asn Trp Lys Ser Phe Phe Gly Gly
                                                              540
TCA GCT TGG ACT TTT GAT GAA ACT ACA AAT GAA TTT TAC CTC CGT TTG TTT GCG AGT CCT
Ser Ala Trp Thr Phe Asp Glu Thr Thr Asn Glu Phe Tyr Leu Arg Leu Phe Ala Ser Arg
                                                              600
CAA GTT GAC TTG AAT TGG GAG AAT GAA GAC TGC AGA AGG GCA ATC TTT GAA AGT CCT GTT
Gln Val Asp Leu Asn Trp Glu Asn Glu Asp Cys Arg Arg Ala Ile Phe Glu Ser Pro Val
                                                              660
GGA TTT TGG CTG GAC CAT GGT GTA GAT GGT TTT AGA ATC GAT ACC GCT GGT TTG TAT TCG
Gly Phe Trp Leu Asp His Gly Val Asp Gly Phe Arg Ile Asp Thr Ala Gly Leu Tyr Ser
                                                              720
AAA CGT CCT GGT TTA CCA GAT TCC CCA ATT TTT GAC AAA ACC TCG AAA TTA CAA CAT CCA
Lys Arg Pro Gly Leu Pro Asp Ser Pro Ile Phe Asp Lys Thr Ser Lys Leu Gln His Pro
                                                              780
AAT TGG GGG TCT CAC AAT GGT CCT AGG ATT CAT GAA TAT CAT CAA GAA CTA CAC AGA TTT
Asn Trp Gly Ser His Asn Gly Pro Arg Ile His Glu Tyr His Gln Glu Leu His Arg Phe
                                                              840
ATG AAA AAC AGG GTG AAA GAT GGT AGA GAA ATA ATG ACA GTC GGT GAA GTT GCC CAT GCA
Met Lys Asn Arg Val Lys Asp Gly Arg Glu Ile Met Thr Val Gly Glu Val Ala His Gly
                                                              900
AGT GAT AAT GCT TTA TAC ACC AGT GCA GCT AGA TAC GAA GTC AGC GAA GTT TTC TCC TTC
Ser Asp Asn Ala Leu Tyr Thr Ser Ala Ala Arg Tyr Glu Val Ser Glu Val Phe Ser Phe
                                                              960
ACG CAC GTT GAA GTT GGT ACC TCG CCA TTT TTC CGT TAT AAC ATA GTG CCC TTC ACC TTG
Thr His Val Glu Val Gly Thr Ser Pro Phe Phe Arg Tyr Asn Ile Val Pro Phe Thr Leu
                                                              1020
AAA CAA TGG AAA GAA GCC ATT GCA TCG AAC TTT TTG TTC ATT AAC GGT ACT GAT AGT TGG
Lys Gln Trp Lys Glu Ala Ile Ala Ser Asn Phe Leu Phe Ile Asn Gly Thr Asp Ser Trp
                                                              1080
GCT ACC ACC TAC ATC GAG AAT CAC GAT CAA GCC CGG TCA ATT ACG AGA TTT GCT GAC GAT
Ala Thr Thr Tyr Ile Glu Asn His Asp Gln Ala Arg Ser Ile Thr Arg Phe Ala Asp Asp
                                                              1140
TCG CCA AAG TAC CGT AAA ATA TCT GGT AAG CTG TTA ACA TTG CTA GAA TGT TCA TTG ACA
Ser Pro Lys Tyr Arg Lys Ile Ser Gly Lys Leu Leu Thr Leu Leu Glu Cys Ser Leu Thr
                                                              1200
GGT ACG TTG TAT GTC TAT CAA GGT CAG GAG ATA GGC CAG ATC AAT TTC AAG GAA TGG CCT
Gly Thr Leu Tyr Val Tyr Gln Gly Gln Glu Ile Gly Gln Ile Asn Phe Lys Glu Trp Pro
                                                              1260
ATT GAA AAG TAT GAG GAC GTT GAT GTG AAA AAC AAC TAC GAG ATT ATC AAA AAA AGT TTT
Ile Glu Lys Tyr Glu Asp Val Asp Val Lys Asn Asn Tyr Glu Ile Ile Lys Lys Ser Phe
                                                              1320
GGT AAA AAC TCG AAG GAA ATG AAG GAT TTT TTT AAA GGA ATC GCC CTA CTT TCT AGA GAT
Gly Lys Asn Ser Lys Glu Met Lys Asp Phe Phe Lys Gly Ile Ala Leu Leu Ser Arg Asp
                                                              1380
CAT TCG AGA ACT CCC ATG CCA TGG ACG AAA GAT AAG CCC AAT GCT GGA TTT ACT GGC CCA
His Ser Arg Thr Pro Met Pro Trp Thr Lys Asp Lys Pro Asn Ala Gly Phe Thr Gly Pro
                                                              1440
GAT GTT AAA CCT TGG TTT TTC TTG AAT GAA TCT TTT GAG CAA GGA ATC AAT GTT GAG CAG
Asp Val Lys Pro Trp Phe Phe Leu Asn Glu Ser Phe Glu Gln Gly Ile Asn Val Glu Gln
                                                              1400
GAA TCC AGA GAT GAT GAC TCA GTT CTC AAT TTT TGG AAA AGG GCC TTG CAA GCC AGA AAG
Glu Ser Arg Asp Asp Asp Ser Val Leu Asn Phe Trp Lys Arg Ala Leu Gln Ala Arg Lys
                                                              1560
AAA TAT AAG GAA GTT ATG ATT TAT GGT TAC GAT TTC CAA TTC ATT GAT TTA GAC AGT GAC
Lys Tyr Lys Glu Val Met Ile Tyr Gly Tyr Asp Phe Gln Phe Ile Asp Leu Asp Ser Asp
                                                              1620
CAG ATC TTT AGC TTC ACT AAA GAG TAC GGA GAC AAG ACG CTG TTT GCT GGT TTG AAT TTC
Gln Ile Phe Ser Phe Thr Lys Glu Tyr Gly Asp Lys Thr Leu Phe Ala Ala Leu Asn Phe
                                                              1680
AGT GGC GAA GAA ATT GAA TTC AGC CTC CCA AGA GAA GGT GCT TCT TTA TCT TTT ATT CTT
Ser Gly Glu Glu Ile Glu Phe Ser Leu Pro Arg Glu Gly Ala Ser Leu Ser Phe Ile Leu
                                                              1740
GGA AAT TAT GAT GAT ACT GAC GTT TCC TCC AGA GTT TTG AAA CCA TGG GAA GGT AGA ATC
Gly Asn Tyr Asp Asp Thr Asp Val Ser Ser Arg Val Leu Lys Pro Trp Glu Gly Arg Ile

TAC CTC GTC AAA TAA
Tyr Leu Val Lys End
```

16

# FIG.2

EP 0 300 425 B1

**YRp/GLUCPI 10,9 Kb** — Hind III, E, Hind III (BamHI), E, F, E, α-GLUCPI, Bcl I, SphI, URA 3, (PvuII), ori, Amp$^r$, TRPI, E

EcoRI — E, F, E, FnuDII — E, F
EcoRI — BclI — E

EcoRI     BclI
AATTCCATGACTATTTCT
GGTACTGATAAAGACTAG

EcoRI
SmaI

**pKK177-3/GLUCPI 4,6 Kb** — PvuI, T2, T1, 5S, EcoRI, α-GLUCPI, Ptac, Bcl I, EcoRI, BamHI, ori, Amp$^r$

**pKK177-3 2,9 Kb** — Amp$^r$, ori, PvuI, Ptac, 5S T1 T2, SmaI, EcoRI, BamHI, (PvuII)

tac     lac operator

| -35 | | -10 | | | RBS | |
|---|---|---|---|---|---|---|
| TTGACA | ATTAATCATCGGCTCG | TATAAT | GTGTGGAATTGTGAGCGGATAACAATTTCACAC | | AGGA | AACAGAATTCC ATG |